(19) Europäisches Patentamt
European Patent Office
Office européen des brevets

(11) Publication number: **0 436 734 B1**

# (12) EUROPEAN PATENT SPECIFICATION

(45) Date of publication of patent specification: **11.10.95**

(51) Int. Cl.6: **C07D 295/32**, A61K 31/495

(21) Application number: **90910946.4**

(22) Date of filing: **12.07.90**

(86) International application number:
**PCT/JP90/00898**

(87) International publication number:
**WO 91/01979 (21.02.91 91/05)**

## (54) NEW AMINOPIPERAZINE DERIVATIVES.

(30) Priority: **02.08.89 GB 8917687**

(43) Date of publication of application:
**17.07.91 Bulletin 91/29**

(45) Publication of the grant of the patent:
**11.10.95 Bulletin 95/41**

(84) Designated Contracting States:
**CH DE DK FR GB IT LI SE**

(56) References cited:
**GB-A- 722 627**
**JP-A-61 106 570**
**JP-B- 3 919 175**

**CA 108 (5): 37783g (Acta Pol. pharm., 43(5), 513-15 (1986), Bulacinski et al.)**

(73) Proprietor: **FUJISAWA PHARMACEUTICAL CO., LTD.**
**4-7, Doshomachi 3-chome**
**Chuo-ku**
**Osaka-shi**
**Osaka 541 (JP)**

(72) Inventor: **OKU, Teruo**
**17-1, Umezono 2-chome**
**Tsukuba-shi,**
**Ibaraki 305 (JP)**
Inventor: **TODO, Eishiro**
**1-8-302, Kitamidorigaoka 2-chome**
**Toyonaka-shi,**
**Osaka 560 (JP)**
Inventor: **YOKOTA, Yoshihiro**
**2-1, Umezono 2-chome**
**Tsukuba-shi,**
**Ibaraki 305 (JP)**
Inventor: **KAYAKIRI, Hiroshi**
**5-4-506, Umezono 2-chome**
**Tsukuba-shi, Ibaraki 305 (JP)**
Inventor: **HASHIMOTO, Masashi**
**21-6-17, Nakayama-satsukidai**
**Takarazuka-shi,**
**Hyogo 665 (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 436 734 B1

(74) Representative: **Türk, Dietmar, Dr. rer. nat.**
**Türk, Gille, Hrabal, Leifert**
**Patentanwälte**
**Brucknerstrasse 20**
**D-40593 Düsseldorf (DE)**

## Description

TECHNICAL FIELD

This invention relates to new aminopiperazine derivatives and pharmaceutically acceptable salts thereof which are useful as a medicament.

BACKGROUND ART

Some piperazine derivatives have been known as useful anti-amnesia agents, for example, in G.B. Patent Application Publication No. 2 162 843 and EP Patent Application Publication No. 299 493, but the N-substituents of piperazine ring in these compounds do not contain amino group while our compounds are N-aminopiperazine derivatives.

Furthermore, GB-A-722,627 discloses 4-substituted-1-aminopiperazine derivatives, the substituent on 4-position of which is alkyl, aralkyl, monocyclic aryl, carbalkoxy, dialkylcarbamoyl or heterocyclic radical, and GB-A-996,253 also discloses 4-substituted-1-aminopiperazine derivatives, the substituent on 4-position of which is alkyl, hydroxyalkyl, benzyl, ar-methylbenzyl, ar-methoxybenzyl, ar-halobenzyl, benzhydryl or ar-chlorobenzhydryl. Moreover, it is not disclosed that 4-substituted-1-aminopiperazine derivatives in GB-A-722,627 and GB-A-996,253 are useful as an anti-amnesia agent or anti-dementia agent.

DISCLOSURE OF INVENTION

This invention relates to new aminopiperazine derivatives and pharmaceutically acceptable salts thereof.

More particularly, it relates to new aminopiperazine derivatives and pharmaceutically acceptable salts thereof which have the potentiation of the cholinergic activity, to processes for the preparation thereof, to a pharmaceutical composition comprising the same, and to a method for the treatment of disorders in the central nervous system for human beings, and more particularly to method for the treatment of amnesia, dementia and senile dementia.

One object of this invention is to provide new and useful aminopiperazine derivatives and pharmaceutically acceptable salts thereof which possess the potentiation of the cholinergic activity.

Another object of this invention is to provide processes for preparation of said aminopiperazine derivatives and salts thereof.

A further object of this invention is to provide a pharmaceutical composition comprising, as an active ingredient, said aminopiperazine derivatives and pharmaceutically acceptable salts thereof.

Still further object of this invention is to provide a therapeutic method for the treatment of disorders in the central nervous system, for human beings, and more particularly of amnesia, dementia and senile dementia, using said aminopiperazine derivatives and pharmaceutically acceptable salts thereof.

The aminopiperazine derivatives of this invention are new and can be represented by the following general formula [I] :

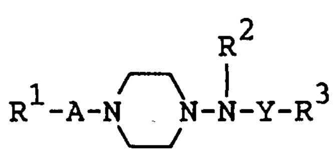

[I]

wherein

- $R^1$ is $C_1$-$C_6$ alkyl, aryl, ar($C_1$-$C_6$)alkoxy or a heterocyclic group, each of which may be substituted with halogen,
- $R^2$ is hydrogen or $C_1$-$C_6$ alkyl,
- $R^3$ is cyclopropyl, cyclobutyl, cyclopentyl, cyclohexyl or aryl, each of which may be substituted with halogen,
- A is

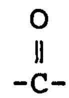

or $-SO_2-$, and

Y is

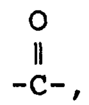

$-SO_2-$ or

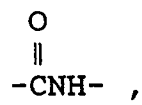

and pharmaceutically acceptable salts thereof.

The object compound [I] or its salt can be prepared by processes as illustrated in the following reaction schemes.

Process 1

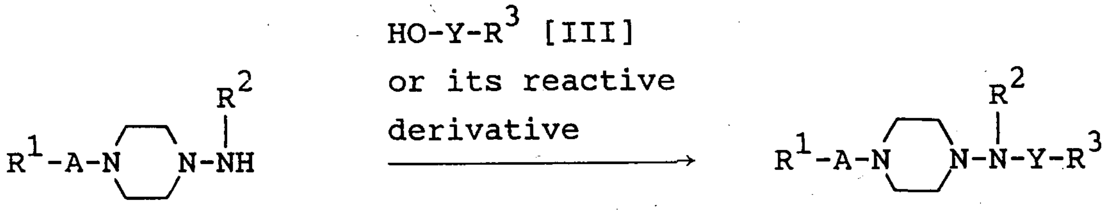


[II]
or its salt

[I]
or its salt

Process 2

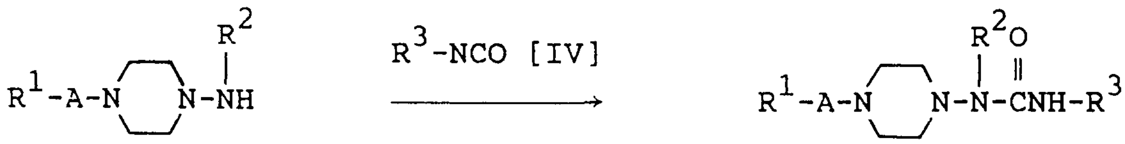


[II] or its salt

[Ia] or its salt

Process 3

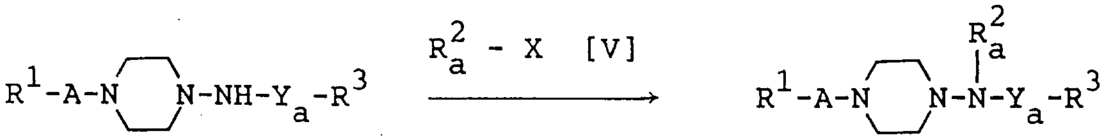


[Ib] or its salt

[Ic]

wherein

$R^1$, $R^2$, $R^3$, A and Y are each as defined above,

$R_a^2$ is $C_1$-$C_6$ alkyl,

$Y_a$ is

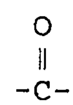

or $-SO_2-$, and

X is an acid residue.

In the above and subsequent description of the present specification, suitable examples of the various definitions to be included within the scope of the invention are explained in detail in the following.

The term "$C_1$-$C_6$" is intended to mean a group having 1 to 6 carbon atom(s), unless otherwise provided.

Suitable "$C_1$-$C_6$ alkyl" may be a straight or branched one such as methyl, ethyl, propyl, isopropyl, butyl, isobutyl, tert-butyl, pentyl and hexyl, in which preferable one is methyl.

Suitable "aryl" may be phenyl, naphthyl, tolyl, xylyl, mesityl and cumenyl, in which preferable one is phenyl or naphthyl.

Suitable "ar($C_1$-$C_6$)alkoxy" may be benzyloxy, phenethyloxy, phenylpropoxy, benzhydryloxy and trityloxy.

Suitable "heterocyclic group" may include saturated or unsaturated, monocyclic or polycyclic one containing at least one hetero atom such as nitrogen atom, oxygen atom or sulfur atom.

The preferred examples of thus defined "heterocyclic group" may be unsaturated, 3 to 8-membered, more preferably 5 or 6-membered heteromonocyclic group containing 1 to 4-nitrogen atom(s), for example, pyrrolyl, imidazolyl, pyrazolyl, pyridyl, pyridyl N-oxide, dihydropyridyl, tetrahydropyridyl, pyrimidyl, pyrazinyl, pyridazinyl, triazinyl, triazolyl, tetrazinyl and tetrazolyl;

unsaturated, condensed heterocyclic group containing 1 to 5 nitrogen atom(s), for example, indolyl, isoindolyl, indolizinyl, benzimidazolyl, quinolyl, isoquinolyl, indazolyl and benzotriazolyl;

unsaturated, 3 to 8-membered heteromonocyclic group containing 1 to 2 oxygen atom(s) and 1 to 3 nitrogen atom(s), for example, oxazolyl, isoxazolyl and oxadiazolyl;

saturated, 3 to 8-membered heteromonocyclic group containing 1 to 2 oxygen atom(s) and 1 to 3 nitrogen atom(s), for example, morpholino and sydnonyl;

unsaturated, condensed heterocyclic group containing 1 to 2 oxygen atom(s) and 1 to 3 nitrogen atom(s), for example, benzoxazolyl and benzoxadiazolyl;

unsaturated, 3 to 8-membered heteromonocyclic group containing 1 to 2 sulfur atom(s) and 1 to 3 nitrogen atom(s), for example, thiazolyl, isothiazolyl and thiadiazolyl;

unsaturated, 3 to 8-membered heteromonocyclic group containing 1 to 2 sulfur atom(s), for example, thienyl;

unsaturated, condensed heterocyclic group containing 1 to 2 sulfur atom(s) and 1 to 3 nitrogen atom(s), for example, benzothiazolyl and benzothiadiazolyl;

unsaturated, 3 to 8-membered heteromonocyclic group containing an oxygen atom, for example, furyl;

unsaturated, condensed heterocyclic group containing 1 to 2 sulfur atom(s), for example, benzothienyl;

unsaturated, condensed heterocyclic group containing 1 to 2 oxygen atom(s), for example, benzofuranyl.

Suitable "acid residue" may include halogen (e.g. fluorine, chlorine, bromine and iodine), arenesulfonyloxy (e.g., benzenesulfonyloxy and tosyloxy), $C_1$-$C_6$ alkanesulfonyloxy (e.g. mesyloxy and ethanesulfonyloxy).

The above-mentioned "$C_1$-$C_6$ alkyl", "aryl", "ar($C_1$-$C_6$) alkoxy", "heterocyclic group", "cyclopropyl", "cyclobutyl", "cyclopentyl" and "cyclohexyl" may be substituted with halogen [e.g. fluorine, chlorine, bromine and iodine].

Suitable pharmaceutically acceptable salts of the object compound [I] are conventional non-toxic salts and include acid addition salt such as an inorganic acid addition salt leg. hydrochloride, hydrobromide, sulfate and phosphate], an organic acid addition salt [e.g. formate, acetate, trifluoroacetate, maleate, tartrate, methanesulfonate, benzenesulfonate and toluenesulfonate], a salt with an amino acid [e.g. aspartic acid salt and glutamic acid salt].

The processes for preparing the object compound [I] are explained in detail in the following.

Process 1

The compound [I] or its salt can be prepared by reacting a compound [II] or its salt with a compound [III] or its reactive derivative at the carboxy or sulfo group.

Suitable salts of the compound [II] may be the same as those exemplified for the compound [I].

Suitable reactive derivative at the carboxy or sulfo group of the compound [III] may include an ester, an acid halide and an acid anhydride. The suitable examples of the reactive derivatives may be an acid halide [e.g. acid chloride and acid bromide]; a symmetrical acid anhydride; a mixed acid anhydride with an acid such as aliphatic carboxylic acid [e.g. acetic acid and pivalic acid], substituted phosphoric acid [e.g. dialkylphosphoric acid and diphenylphosphoric acid];
an ester such as substituted or unsubstituted $C_1$-$C_6$ alkylester [e.g. methyl ester, ethyl ester, propyl ester, hexyl ester and trichloromethyl ester], substituted or unsubstituted ar($C_1$-$C_6$)alkyl ester [e.g. benzyl ester, benzhydryl ester and p-chlorobenzyl ester], substituted or unsubstituted aryl ester [e.g. phenyl ester, tolyl ester, 4-nitrophenyl ester, 2,4-dinitrophenyl ester, pentachlorophenyl ester and naphthyl ester], or an ester with N,N-dimethylhydroxylamine, N-hydroxysuccinimide, N-hydroxyphthalimide or 1-hydroxy-6-chloro-1H-benzotriazole. These reactive derivatives can be optionally selected according to the kind of the compound [III] to be used.

The reaction is usually carried out in a conventional solvent such as water, acetone, dioxane, chloroform, methylene chloride, ethylene chloride, tetrahydrofuran, ethyl acetate, N,N-dimethylformamide, pyridine or any other organic solvent which does not adversely influence the reaction. Among these solvents, hydrophilic solvents may be used in a mixture with water.

When the compound [III] is used in a free acid form or its salt form in the reaction, the reaction is preferably carried out in the presence of a conventional condensing agent such as N,N'-dicyclohexylcarbodiimide, N-cyclohexyl-N'-morpholinoethylcarbodiimide, N-ethyl-N'-(3-dimethylaminopropyl)carbodiimide, thionyl chloride, oxalyl chloride, $C_1$-$C_6$ alkoxycarbonyl halide [e.g. ethyl chloroformate and isobutyl chloroformate] and 1-(p-chlorobenzenesulfonyloxy) -6-chloro-1H-benzotriazole.

The reaction temperature is not critical, and the reaction can be carried out under cooling to heating.

Process 2

The compound [Ia] or its salt can be prepared by reacting a compound [II] or its salt with a compound [IV].

Suitable salts of the compound [II] may be the same as those exemplified for the compound [I].

This reaction is usually carried out in a solvent such as dioxane, tetrahydrofuran, benzene, chloroform, methylene chloride or any other organic solvent which does not adversely influence the reaction.

The reaction temperature is not critical, and the reaction is usually carried out under cooling to warming.

Process 3

The compound [Ic] can be prepared by reacting a compound [Ib] or its salt with a compound [V].

Suitable salts of the compound [Ib] may be the same as those exemplified for the compound [I].

The present reaction is preferably carried out in the presence of base such as an alkali metal (e.g. lithium, sodium and potassium), alkaline earth metal (e.g. calcium), alkali metal hydride (e.g. sodium hydride) and alkaline earth metal hydride (e.g. calcium hydride).

This reaction is usually carried out in a solvent such as N,N-dimethylformamide, diethyl ether, tetrahydrofuran, dioxane, benzene, toluene or any other solvent which does not adversely influence the reaction.

The reaction temperature is not critical, and the reaction is usually carried out under cooling to heating.

The compounds obtained by the above processes can be isolated and purified by a conventional method such as pulverization, recrystallization, column chromatography and reprecipitation.

The object compounds [I] and pharmaceutically acceptable salts thereof possess strong potentiation of the cholinergic activity, and are useful for the treatment of disorders in the central nervous system for human beings, and more particularly of amnesia, dementia and senile dementia.

In order to illustrate the usefulness of the object compound [I], the pharmacological test data of the compound [I] are shown in the following.

Anti-scopolamine-induced amnesia activity

Test Method :

Male Wistar rats (body weight 250-300 g, 23-31 per group) were used. The effect of the test compound on deficit of memory induced by scopolamine (a muscarinic acetylcholine receptor antagonist) in passive avoidance task was examined. The apparatus consists of an illuminated compartment attached to a dark compartment equipped with a grid floor. These compartments are separated with a guillotine door. The rat was placed in the illuminated compartment and 10 seconds after placing the rat, the guillotine door was raised. After the rat entered into the dark compartment, the rat was returned to its home cage (Habituation). 30 minutes after habituation, scopolamine hydrobromide (1 mg/kg) (Sigma Co. St Louis, MO, U.S.A.) was intraperitoneally (i.p.) administered. 60 minutes after habituation, the acquisition trial was carried out. The rat was again placed in the illuminated chamber. When the rat entered in the dark chamber, the guillotine door was closed and a 4mA scrambled electrical footshock was delivered for 3 seconds through the grid floor. Test compounds were intraperitoneally administered immediately after this training.

In the test trial given 24 hours after the training, the rat was placed again in the illuminated compartment and the response latency to enter into the dark compartment was measured to an extent of 300 seconds at the longest. Results were recorded as the mean latency of step through for each experimental group of rats. The effect of the test compound was presented as a percentage of the recovery. The calculation was based on the following equation.

$$\text{Percentage of Recovery (\%)} = \frac{A - B}{300 - B} \times 100$$

A : mean latency of test compound (sec)

B : mean latency of vehicle (sec)

Test Compounds :

(a) N-(4-Acetyl-1-piperazinyl)-4-fluorobenzamide
(b) N-(4-Benzoyl-1-piperazinyl)-4-fluorobenzamide
(c) N-(4-Acetyl-1-piperazinyl)-4-fluorobenzenesulfonamide

Test Results :

Percentage of Recovery (%)

| Dose (mg/kg) \ Test Compound | (a) | (b) | (c) |
|---|---|---|---|
| 0 | 0 | 0 | 0 |
| 0.1 | 23.0 | 26.4 | 20.4 |
| 0.32 | 24.6 | 29.5 | 26.8 |
| 1.0 | 40.3 | 19.7 | 34.5 |
| 3.2 | 39.2 | 41.5 | 50.2 |

For therapeutic purpose, the compound [I] and a pharmaceutically acceptable salt thereof of the present invention can be used in a form of pharmaceutical preparation containing one of said compounds, as an active ingredient, in admixture with a pharmaceutically acceptable carrier such as an organic or inorganic solid or liquid excipient suitable for oral, parenteral or external administration. The pharmaceutical preparations may be capsules, tablets, dragees, granules, solution, suspension or emulsion. If desired, there may be included in these preparations, auxiliary substances, stabilizing agents, wetting or emulsifying agents, buffers and other commonly used additives.

While the dosage of the compound [I] ,will vary depending upon the age and condition of the patient, an average single dose of about 0.1 mg, 1 mg, 10 mg, 50 mg, 100 mg, 250 mg, 500 mg and 1000 mg of the compound [I] may be effective for treating the above-mentioned diseases. In general, amounts between 0.1 mg/body and about 1,000 mg/body may be administered per day.

The following Preparations and Examples are given for the purpose of illustrating this invention.

Preparation 1

To a mixture of 1-nitrosopiperazine (1.21 g) and pyridine (2.4 ml) in dioxane (18 ml) was added dropwise 2-thenoyl chloride (1.2 ml) in an ice bath. The mixture was stirred for one hour in an ice bath and then overnight at ambient temperature. The mixture was poured into a mixture of ethyl acetate (100 ml) and water (100 ml). The separated aqueous layer was extracted twice with ethyl acetate. The combined organic layers were dried and concentrated in vacuo. The residue was pulverized with diisopropyl ether to yield 1-nitroso-4-(2-thenoyl)-piperazine (1.78 g).

To a solution of 1-nitroso-4-(2-thenoyl)piperazine (1.78 g) in a mixture of acetic acid (8 ml) and water (8 ml) was added Zn powder (1.55 g) in several portions at 6-15 °C. The suspension was stirred for 1.5 hours below 10 °C. To the mixture was added acetic acid (4 ml) and Zn powder (780 mg) at 8-12 °C. The mixture was stirred for 2 hours at ambient temperature. To the suspension was added 17% aqueous sodium hydroxide solution (70 ml). The insoluble materials were filtered off. The aqueous layer was extracted with dichloromethane. The combined organic layers were dried and concentrated in vacuo. The residue was purified by column chromatography on silica gel (elution by 5% methanol in dichloromethane) to yield 1-amino-4-(2-thenoyl)piperazine (1.17 g) as a pale yellow syrup.

$^1$H NMR ($CDCl_3$, $\delta$) : 7.48 (1H, dd, J=1 and 5Hz), 7.31 (1H, dd, J=1 and 4Hz), 7.07 (1H, dd, J=4 and 5Hz), 3.83 (4H, t, J=5Hz), 2.88 (2H, br.s), 2.70 (4H, t, J=5Hz)

The following compound (Preparation 2) was obtained according to a similar manner to that of Preparation 1.

Preparation 2

1-Amino-4-(2-naphthoyl)piperazine
pale yellow syrup
$^1$H NMR ($CDCl_3$, $\delta$) : 7.98-7.83 (4H, m), 7.65-7.45 (3H, m), 3.88 (2H, br.s), 3.62 (2H, br.s), 3.04 (2H, br.s), 2.68 (4H, br.s)

Example 1

To a mixture of 1-acetyl-4-aminopiperazine dihydrochloride (540 mg) and 4-fluorobenzenesulfonyl chloride (535 mg) in dichloromethane (5 ml) was added triethylamine (1.2 ml) in a few minutes with stirring in an ice bath. After stirring for half an hour at the same temperature, the mixture was washed with brine, dried, and concentrated in vacuo. The residue was crystallized from ethanol and recrystallized from a mixture of ethanol and diethyl ether to yield N-(4-acetyl-1-piperazinyl)-4-fluorobenzenesulfonamide (360 mg) as white crystals.
mp : 185-186 °C
IR ($CHCl_3$) : 3400, 3300, 3005, 1642, 1638, 1596, 1498, 1460, 1256, 1240, 1170, 1155, 1094 $cm^{-1}$

Example 2

To a solution of 1-amino-4-benzoylpiperazine (1.1 g) and triethylamine (0.6 g) in dichloromethane (20 ml) was added dropwise 4-fluorobenzoyl chloride (0.86 g) with stirring in an ice bath. After stirring for one hour at the same temperature, the mixture was washed with 1N hydrochloric acid, dried, and concentrated in vacuo. The residue was crystallized from diisopropyl ether and purified by recrystallization from ethanol to give N-(4-benzoyl-1-piperazinyl)-4-fluorobenzamide (0.97 g) as white crystals.
mp : 206-207 °C
$^1$H NMR ($CDCl_3$, $\delta$) : 7.78 (2H, dd, J = 5 and 9Hz), 7.43 (5H, s), 7.34 (1H, br.s), 7.12 (2H, t, J = 9Hz), 3.96 (2H, br.s), 3.67 (2H, br.s), 3.03 (4H, br.s)
The following compound (Example 3) was obtained according to a similar manner to that of Example 1.

Example 3

N-(4-Benzenesulfonyl-1-piperazinyl)-4-fluorobenzenesulfonamide
mp : 190-191 °C
$^1$H NMR ($CDCl_3$-$CD_3OD$ = 1:1, $\delta$) : 7.88 (2H, dd, J = 5 and 9Hz), 7.78-7.57 (5H, m), 7.20 (2H, t, J = 9Hz), 3.03-2.92 (4H, m), 2.75-2.66 (4H, m)
The following compounds (Examples 4-l) to 4-9)) were obtained according to a similar manner to that of Example 2.

Example 4

1) N-(4-Acetyl-1-piperazinyl)-2-fluorobenzamide
mp : 171-172 °C
IR ($CHCl_3$) : 3480, 3400, 3015, 1678, 1644, 1619, 1488, 1458, 1444, 1300, 1285, 1262 $cm^{-1}$
2) N-(4-Acetyl-1-piperazinyl)-3-fluorobenzamide
mp : 196-197 °C
IR ($CHCl_3$) : 3350, 3005, 1680, 1640, 1592, 1440, 1282, 1258 $cm^{-1}$
3) N-(4-Acetyl-1-piperazinyl)-4-fluorobenzamide
mp : 211-212 °C
$^1$H NMR (DMSO-$d_6$, $\delta$) : 9.58 (1H, s), 7.84 (2H, dd, J = 6 and 9Hz), 7.31 (2H, t, J = 9Hz), 3.52 (4H, t, J = 6Hz), 2.88 (2H, t, J = 6Hz), 2.82 (2H, t, J = 6Hz), 2.03 (3H, s)
4) N-(4-Acetyl-1-piperazinyl)cyclohexanecarboxamide
mp : 220-221 °C
IR ($CHCl_3$) : 3430, 3340, 2998, 2940, 2852, 1638, 1448, 1280, 1256, 1140, 1118, 998 $cm^{-1}$

5) N-(4-Phenylsulfonyl-1-piperazinyl)-4-fluorobenzamide
mp : 274-276 °C
$^1H$ NMR ($CDCl_3$-$CD_3OD$ = 1:1, δ) : 7.86-7.58 (7H, m), 7.14 (2H, t, J = 9Hz), 3.28-3.18 (4H, m), 3.07-2.98 (4H, m)
6) N-(4-Methylsulfonyl-1-piperazinyl)-4-fluorobenzamide
mp : 257-258 °C
$^1H$ NMR ($CDCl_3$-$CD_3OD$ = 1:1, δ) : 7.80 (2H, dd, J = 5 and 9Hz), 7.12 (2H, t, J = 9Hz), 3.40 (4H, t, J = 5Hz), 2.99 (4H, t, J = 5Hz), 2.88 (3H, s)
7) N-[4-(2-Thenoyl)-1-piperazinyl]-4-fluorobenzamide
mp : 189-190 °C
IR ($CHCl_3$) : 3440, 3340, 3280, 3000, 1674, 1604, 1501, 1438, 1284, 1259 $cm^{-1}$
8) N-[4-(2-Naphthoyl)-1-piperazinyl]-4-fluorobenzamide
mp : 227-228 °C
IR ($CHCl_3$) : 3430, 3335, 2990, 1668, 1617, 1601, 1496, 1477, 1438, 1284, 1260 $cm^{-1}$
9) N-(4-Benzyl-1-piperazinyl)-4-fluorobenzamide
mp : 165-168 °C
$^1H$ NMR ($CDCl_3$, δ) : 7.77 (2H, dd, J = 5 and 9Hz), 7.45-7.23 (5H, m), 7.10 (2H, t, J = 9Hz), 3.41 (2H, s), 3.00 (4H, br.s), 2.72 (4H, br.s)

Example 5

To a solution of 1-acetyl-4-aminopiperazine dihydrochloride (540 mg) in dichloromethane (5 ml) was added dropwise triethylamine (0.8 ml) followed by addition a solution of 4-fluorophenylisocyanate (377 mg) in dichloromethane (2.5 ml) with stirring in an ice bath. After stirring for 100 minutes at the same temperature, a mixture of dichloromethane (15 ml) and methanol (3 ml) was added to the reaction mixture. The mixture was washed with brine, dried, and concentrated in vacuo. The residue was crystallized from ethanol to yield N-(4-acetyl-1-piperazinyl)-N'-(4-fluorophenyl)urea (500 mg).

mp : 210-211 °C

$^1H$ NMR ($CDCl_3$, δ) : 7.98 (1H, s), 7.40 (2H, dd, J = 5 and 9Hz), 7.00 (2H, t, J = 9Hz), 5.88 (1H, br.s), 3.80-2.50 (8H, m) 2.14 (3H, s)

Example 6

To a solution of N-(4-acetyl-1-piperazinyl)-4-fluorobenzamide (769 mg) in N,N-dimethylformamide (8 ml) was added sodium hydride (116 mg, 60% oil dispersion) in one portion with stirring in an ice bath. The mixture was stirred for one hour at the same temperature and therein iodomethane (0.3 ml) was added. After stirring for one hour in an ice bath, the mixture was concentrated in vauco. The residue was purified by column chromatography on Dowex (50W x 8, $H^+$) to give N-(4-acetyl-1-piperazinyl)-N-methyl-4-fluorobenzamide (440 mg).

mp : 205-206 °C

IR ($CHCl_3$) : 3010, 2960, 1652, 1612, 1570, 1508, 1440, 1344, 1268, 1154 $cm^{-1}$

**Claims**

**1.** A compound of the formula :

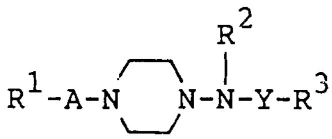

[I]

wherein

$R^1$ is $C_1$-$C_6$ alkyl, aryl, ar($C_1$-$C_6$)alkoxy or a heterocyclic group, each of which may be substituted with halogen,

$R^2$ is hydrogen or $C_1$-$C_6$ alkyl,

$R^3$ is cyclopropyl, cyclobutyl, cyclopentyl, cyclohexyl or aryl, each of which may be substituted

with halogen,

A is

$$-\overset{O}{\overset{\|}{C}}-$$

or $-SO_2-$, and

Y is

$$-\overset{O}{\overset{\|}{C}}-,$$

$-SO_2-$ or

$$-\overset{O}{\overset{\|}{C}}NH-,$$

and pharmaceutically acceptable salts thereof.

**2.** A process for preparing a compound of the formula :

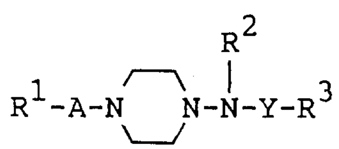

[I]

wherein

$R^1$ is $C_1$-$C_6$ alkyl, aryl, ar($C_1$-$C_6$)alkoxy or a heterocyclic group, each of which may be substituted with halogen,

$R^2$ is hydrogen or $C_1$-$C_6$ alkyl,

$R^3$ is cyclopropyl, cyclobutyl, cyclopentyl, cyclohexyl or aryl, each of which may be substituted with halogen,

A is

$$-\overset{O}{\overset{\|}{C}}-$$

or $-SO_2-$, and

Y is

$$-\overset{O}{\overset{\|}{C}}-,$$

$-SO_2-$ or

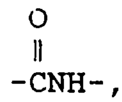

and pharmaceutically acceptable salts thereof, which comprises,

a) reacting a compound of the formula :

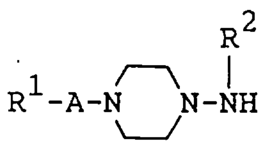

[II]

or its salt with a compound of the formula :

HO-Y-$R^3$ [III]

or its reactive derivative to provide a compound of the formula :

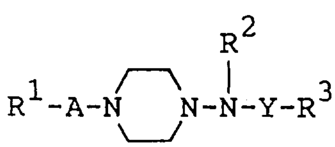

[I]

or its pharmaceutically acceptable salt,

in the above formulas, $R^1$, $R^2$, $R^3$, A and Y are each as defined above,

b) reacting a compound of the formula :

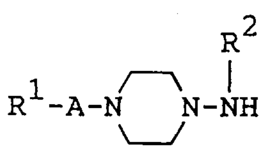

[II]

or its salt with a compound of the formula :

$R^3$-NCO [IV]

to provide a compound of the formula :

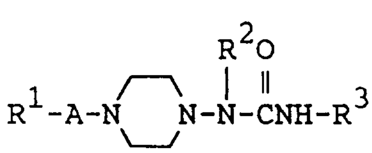

[Ia]

or its pharmaceutically acceptable salt,

in the above formulas, $R^1$, $R^2$, $R^3$ and A are each as defined above, or

c) reacting a compound of the formula :

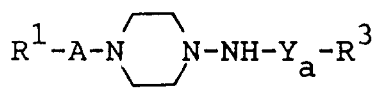 [Ib]

or its salt with a compound of the formula :

$R^2_a$ - X [V]

to provide a compound of the formula :

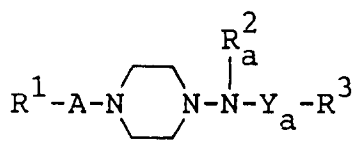 [Ic]

in the above formulas, $R^1$, $R^3$ and A are each as defined above, $R^2_a$ is $C_1$-$C_6$ alkyl, $Y_a$ is

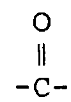

or $-SO_2-$, and X is an acid residue.

**3.** A pharmaceutical composition comprising a compound of claim 1, as an active ingredient, in association with a pharmaceutically acceptable, substantially nontoxic carrier or excipient.

**4.** A compound of claim 1 for use as a medicament.

**5.** A pharmaceutical composition according to claim 3 as a potentiating agent of the cholinergic activity.

**6.** A pharmaceutical composition according to claim 5 as an anti-amnesia agent or anti-dementia agent.

**7.** A compound according to claim 1,
wherein
$R^1$ is $C_1$-$C_6$ alkyl, phenyl, naphthyl or thienyl, and
$R^3$ is phenyl which may be substituted with halogen.

**8.** A compound according to claim 7,
wherein
$R^2$ is hydrogen,
$R^3$ is phenyl which is substituted with halogen, and
Y is

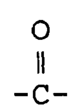

or $-SO_2-$.

**9.** A compound of claim 8, which is N-(4-acetyl-1-piperazinyl)-4-fluorobenzamide.

**10.** A compound of claim 8, which is N-(4-benzoyl-1-piperazinyl)-4-fluorobenzamide.

**11.** A compound of claim 8, which is N-(4-acetyl-1-piperazinyl)-4-fluorobenzenesulfonamide.

**Patentansprüche**

**1.** Verbindung der Formel

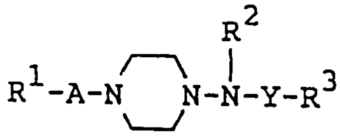

[I]

worin bedeuten:

- $R^1$ $C_1$-$C_6$-Alkyl, Aryl, Ar($C_1$-$C_6$)-alkoxy oder eine heterocyclische Gruppe, von denen jeder Rest durch Halogen substituiert sein kann,
- $R^2$ Wasserstoff oder $C_1$-$C_6$-Alkyl,
- $R^3$ Cyclopropyl, Cyclobutyl, Cyclopentyl, Cyclohexyl oder Aryl, von denen jeder Rest durch Halogen substituiert sein kann,
- A

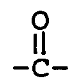

oder $-SO_2-$ und

- Y

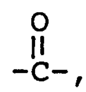

$-SO_2-$ oder

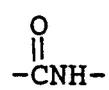

und ihre pharmazeutisch akzeptablen Salze.

**2.** Verfahren zur Herstellung einer Verbindung der Formel

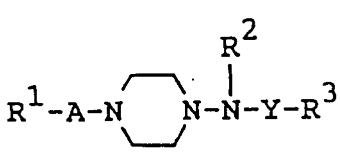

[I]

worin bedeuten:

- $R^1$ $C_1$-$C_6$-Alkyl, Aryl, Ar($C_1$-$C_6$)-alkoxy oder eine heterocyclische Gruppe, von denen jeder Rest durch Halogen substituiert sein kann,

$R^2$ Wasserstoff oder $C_1$-$C_6$-Alkyl,

$R^3$ Cyclopropyl, Cyclobutyl, Cyclopentyl, Cyclohexyl oder Aryl, von denen jeder Rest durch Halogen substituiert sein kann,

A

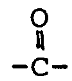

oder $-SO_2-$ und

Y

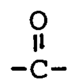,

$-SO_2-$ oder

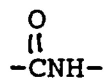

und ihrer pharmazeutisch akzeptablen Salze, das umfaßt

a) die Umsetzung einer Verbindung der Formel

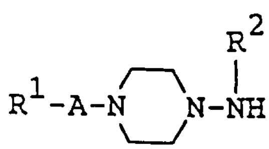

[II]

oder ihres Salzes mit einer Verbindung der Formel

$HO-Y-R^3$ [III]

oder ihres reaktionsfähigen Derivats unter Bildung einer Verbindung der Formel

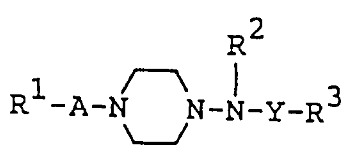

[I]

oder ihres pharmazeutisch akzeptablen Salzes,

wobei in den obengenannten Formeln $R^1$, $R^2$, $R^3$, A und Y jeweils wie oben definiert sind,

b) die Umsetzung einer Verbindung der Formel

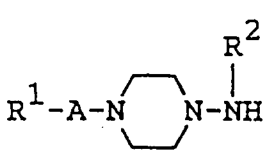

[II]

oder ihres Salzes mit einer Verbindung der Formel

$R^3$-NCO [IV]

unter Bildung einer Verbindung der Formel

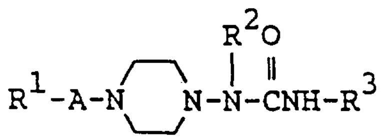

[Ia]

oder ihres pharmazeutisch akzeptablen Salzes,
wobei in den obengenannten Formeln $R^1$, $R^2$, $R^3$ und A jeweils wie oben definiert sind, oder
c) die Umsetzung einer Verbindung der Formel

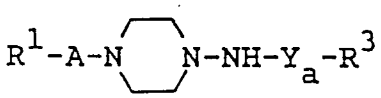

[Ib]

oder ihres Salzes mit einer Verbindung der Formel

$R^2_a$ - X [V]

unter Bildung einer Verbindung der Formel

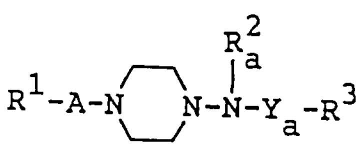

[Ic]

wobei in den obengenannten Formeln $R^1$, $R^3$ und A jeweils wie oben definiert sind, $R^2{}_a$ für $C_1$-$C_6$-Alkyl, $Y_a$ für

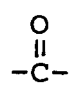

oder -$SO_2$- und X für einen Säurerest stehen.

**3.** Pharmazeutische Zusammensetzung, die eine Verbindung nach Anspruch 1 als einen aktiven Bestandteil (Wirkstoff) in Assoziation (Kombination) mit einem pharmazeutisch akzeptablen, im wesentlichen nicht-toxischen Träger oder Exzipienten enthält.

**4.** Verbindung nach Anspruch 1 für die Verwendung als Arzneimittel.

**5.** Pharmazeutische Zusammensetzung nach Anspruch 3 als Mittel zur Potenzierung (Verstärkung) der cholinergischen Aktivität.

**6.** Pharmazeutische Zusammensetzung nach Anspruch 5 als Antiamnesie-Mittel oder Antidemenz-Mittel.

**7.** Verbindung nach Anspruch 1, worin bedeuten:
$R^1$ $C_1$-$C_6$-Alkyl, Phenyl, Naphthyl oder Thienyl und

$R^3$ Phenyl, das durch Halogen substituiert sein kann.

**8.** Verbindung nach Anspruch 7, worin bedeuten:
$R^2$ Wasserstoff,
$R^3$ Phenyl, das durch Halogen substituiert ist, und
Y

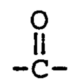

oder $-SO_2-$.

**9.** Verbindung nach Anspruch 8, bei der es sich handelt um N-(4-Acetyl-1-piperazinyl)-4-fluorobenzamid.

**10.** Verbindung nach Anspruch 8, bei der es sich handelt um N-(4-Benzoyl-1-piperazinyl)-4-fluorobenzamid.

**11.** Verbindung nach Anspruch 8, bei der es sich handelt um N-(4-Acetyl-1-piperazinyl)-4-fluorobenzolsulfonamid.

**Revendications**

**1.** Composé répondant à la formule :

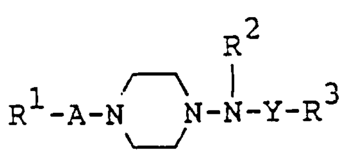

[I]

dans laquelle $R^1$ est un groupe alkyle en $C_1$-$C_6$, aryle, ar(alcoxy en $C_1$-$C_6$) ou un groupe hétérocyclique, dont chacun peut être substitué par un atome d'halogène, $R^2$ est un atome d'hydrogène ou un groupe alkyle en $C_1$-$C_6$,$R^3$ est un groupe cyclopropyle, cyclobutyle, cyclopentyle, cyclohexyle ou aryle, dont chacun peut être substitué par un atome d'halogène,
A est

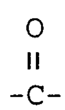

ou $-SO_2-$ et
Y est

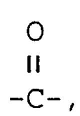

$-SO_2-$ ou

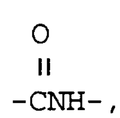

et des sels pharmaceutiquement acceptables de celui-ci.

**2.** Procédé pour préparer un composé répondant à la formule :

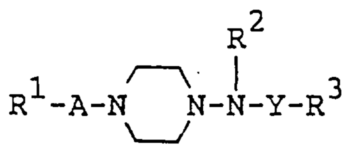

[I]

dans laquelle $R^1$ est un groupe alkyle en $C_1$-$C_6$, aryle, ar(alcoxy en $C_1$-$C_6$) ou un groupe hétérocyclique, dont chacun peut être substitué par un atome d'halogène,
$R^2$ est un atome d'hydrogène ou un groupe alkyle en $C_1$-$C_6$,
$R^3$ est un groupe cyclopropyle, cyclobutyle, cyclopentyle, cyclohexyle ou aryle, dont chacun peut être substitué par un atome d'halogène,
A est

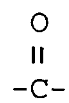

ou $-SO_2-$ et
Y est

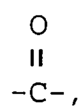

$-SO_2-$ ou

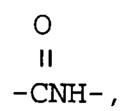

et des sels pharmaceutiquement acceptables de celui-ci, qui consiste à :
a) faire réagir un composé répondant à la formule :

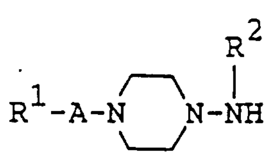

[II]

ou son sel avec un composé répondant à la formule :

$HO-Y-R^3$ [III]

ou son dérivé réactif pour fournir un composé répondant à la formule :

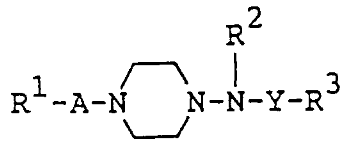 [I]

ou son sel pharmaceutiquement acceptable,
dans les formules ci-dessus, $R^1$, $R^2$, $R^3$, A et Y sont chacun tels que définis ci-dessus,
b) faire réagir un composé répondant à la formule :

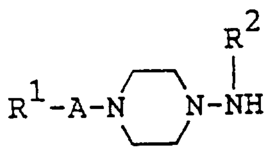 [II]

ou son sel avec un composé répondant à la formule :

$R^3$-NCO [IV]

pour fournir un composé répondant à la formule :

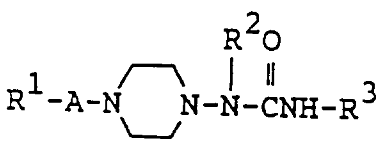 [Ia]

ou son sel pharmaceutiquement acceptable,
dans les formules ci-dessus, $R^1$, $R^2$, $R^3$ et A sont chacun tels que définis ci-dessus, ou
c) faire réagir un composé répondant à la formule :

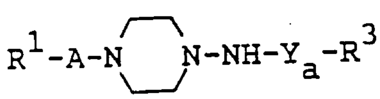 [Ib]

ou son sel avec un composé répondant à la formule :

$R_a^2$ - X [V]

pour fournir un composé répondant à la formule :

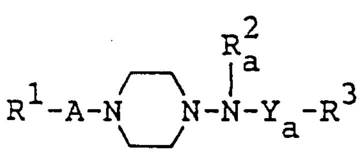 [Ic]

dans les formules ci-dessus, $R^1$, $R^3$ et A sont chacun tels que définis ci-dessus, $R_a^2$ est un groupe alkyle en $C_1$-$C_6$, $Y_a$ est :

$$-\overset{O}{\overset{\parallel}{C}}-$$

ou $-SO_2-$, et X est un résidu acide.

**3.** Composition pharmaceutique comprenant un composé de la revendication 1, comme ingrédient actif, en association avec un support ou excipient pharmaceutiquement acceptable, sensiblement non toxique.

**4.** Composé selon la revendication 1 pour emploi comme médicament.

**5.** Composition pharmaceutique selon la revendication 3, comme agent renforçant l'activité cholinergique.

**6.** Composition pharmaceutique selon la revendication 5 comme agent anti-amnésie ou agent antidémence.

**7.** Composé selon la revendication 1, dans lequel $R^1$ est un groupe alkyle en $C_1$-$C_6$, phényle, naphtyle ou thiényle, et $R^3$ est un groupe phényle qui peut être substitué par un atome d'halogène.

**8.** Composé selon la revendication 7, dans lequel $R^2$ est un atome d'hydrogène, $R^3$ est un groupe phényle qui est substitué par un atome d'halogène, et
Y est

$$-\overset{O}{\overset{\parallel}{C}}-$$

ou $-SO_2-$.

**9.** Composé selon la revendication 8 qui est le N-(4-acétyl-1-pipérazinyl)-4-fluorobenzamide.

**10.** Composé selon la revendication 8, qui est le N-(4-benzoyl-1-pipérazinyl)-4-fluorobenzamide.

**11.** Composé selon la revendication 8, qui est le N-(4-acétyl-1-pipérazinyl)-4-fluorobenzènesulfonamide.